# EUROPEAN PATENT APPLICATION

(11) **EP 1 065 619 A2**
(43) Date of publication of application: **03.01.2001**
(21) Application number: 00305464.0
(22) Date of filing: 29.06.2000
(51) Int. Cl.: G06F 19/00

(54) **Method and apparatus for automatically guiding a user through a medical diagnostic system service workflow**

(30) Priority: 29.06.1999 US 342443
(71) Applicant: GENERAL ELECTRIC COMPANY, Schenectady, NY 12345 (US)
(72) Inventor: Unger, Christopher David, Delafield, Wisconsin 53018 (US); Childress, Frank Willard, Brookfield, Wisconsin 53045 (US)
(74) Representative: Goode, Ian Roy

(57) **Abstract**

Method and apparatus for providing a context sensitive service delivery system for a medical diagnostic system (100). The processor (104) of the medical diagnostic system (100) automatically catalogs system errors into an error log on a storage medium (110) as errors occur. The user is shown a formatted error log on the display (108) of the medical diagnostic system (100). The formatted error log contains hypertext links that each correspond to the first service step of a service workflow associated with the system error. The user uses an input device (106) to select one of the errors to begin the service process with respect to that error. The processor (104) of the medical diagnostic system (100) accesses service information and applications stored on the storage medium (110) and guides the user through one or more service workflows to eliminate the root causes of the selected system error.

## Description

Medical diagnostic systems have become quite commonly used throughout hospitals and clinics as a non-invasive method of examining and diagnosing patients. Examples of medical diagnostic systems include x-ray, ultrasound, magnetic resonance imaging, computed tomography, and nuclear medicine systems. These systems are quite complex and hence require periodic service. Service is also required when systems are upgraded with new features, software, hardware and the like.

In the past, medical diagnostic systems were serviced typically by a service engineer who carried and relied upon multiple volumes of print materials (service manuals) that illustrated a multitude of possible problems and solutions for the particular medical diagnostic system being serviced. Generally, separate service manuals were required for each medical diagnostic system and, often, for each model or version of a particular medical diagnostic system. The service manuals set forth service workflows associated with errors, such as those occurring during operation or installation of a medical diagnostic system. In the simplest sense, a service workflow is a set of possible service paths that lead to the identification and elimination of a root cause of a system error. Past service delivery techniques have experienced many problems.

One problem with past service delivery methods is that companies which manufacture medical diagnostic systems typically have multiple products and product lines. Training costs for service engineers can be very high if the service platform is not consistent across these products and product lines. If the service engineers have a high turn over rate, then this cost can become very high for the company.

Another problem with past service delivery methods is that the service engineer must have numerous volumes of manuals containing service information and storage media containing service applications (e.g., diagnostic and recalibration software) readily available. Service engineers must read numerous pages of information and procedures in order to determine which service steps may be applicable in any given case. Even after a service engineer follows a particular service path through the set of manuals, the engineer may not reach the correct solution. For instance, the engineer may troubleshoot a system error that may be caused by numerous different sources, such as hardware failures, peripheral failures, software faults and the like. The engineer may follow a service path through the manuals to a potentially malfunctioning hardware component, only to find that the hardware component had not failed. Hence, the engineer must backtrack (through the service path within the manuals) to the last point at which there was a choice between multiple possible service paths. Backtracking is difficult and time consuming. It is difficult for the service engineer to maintain the context in which he was performing service steps. Further, it is difficult to keep track of procedures already performed, particularly where multiple service paths were followed in succession that made use of some of the same service steps. In order to provide service in a more efficient manner, a service engineer required a great deal of experience. The service engineer's experience would allow the service engineer to more quickly identify root causes of errors logged in the system. Consequently, a high turnover rate for service engineers (resulting in less efficient service delivery), would lead to increased mean time to repair and higher service costs.

Still another problem is the difficulty in maintaining a current set of service manuals and software applications. Because each service engineer maintains a copy of these service materials, updates must be distributed to each service engineer individually.

A need exists for an improved mechanism for stepping service engineers through the service workflow needed to correct system errors and to update systems. The preferred embodiments of the present invention overcome the problems discussed above, and other concerns heretofore experienced.

According to the present invention, a service delivery method and apparatus are provided for assisting technicians to service medical diagnostic systems more quickly and with less dependence on the technician's experience. The method and apparatus automatically guide a user through a service workflow of a medical diagnostic system. In one example of an application, the medical diagnostic system automatically catalogs system errors into an error log as they occur. The service technician is shown, in the error logging application, a formatted error log containing hypertext links that each correspond to the first service step of a service workflow for an associated system error. The method and apparatus then guide the technician through one or more service paths, such as in a diagnostic application, to eliminate the root causes of the system error.

Initially, historic service data is collected based on past experiences of service engineers, design engineers, field technicians, customer physicians and the like. The historic information is analyzed to construct multiple hierarchical workflow structures. Individual hierarchical workflow structures may be associated with a variety of applications desired for operation in connection with medical diagnostic systems. For instance, individual hierarchical workflow structures may be associated with diagnostics, image quality, calibration, configuration, product maintenance, utilities, part renewal, preventative maintenance, installation of parts, system upgrades, technician guidance applications, error cataloging, and the like. Within any individual application, multiple hierarchical workflow structures may be constructed. For example, a diagnostic application may have one hierarchical workflow structure associated with the troubleshooting process for each error that the system is able to detect.

The initial service step of a hierarchical workflow structure may be reached in various ways. One way is to provide a direct link to a hierarchical workflow structure from the formatted error log. A second possible way to provide access to a hierarchical workflow structure is to link to a service step within it from a service step of another hierarchical workflow structure. A third way in which access may be provided to a hierarchical workflow structure is through a navigational interface (such as an icon bar) that associates each application to a hierarchical workflow structure. An initial service step of a workflow reached by starting an application (for example, by selecting the icon for that application) is an application starting point. Each application starting point has at least one associated service step related thereto. A service step may represent an action carried out by a service engineer during a particular application or a service step may represent an automated operation carried out by the system during interaction with the service engineer to perform service. Each service workflow may include one or more service steps associating one or more final service steps with the initial service step. Based upon historic information about the system, the system constructs one or more hierarchical workflow structures including initial, intermediate and final service steps, as well as service paths. During implementation of a particular service application, the system accesses corresponding hierarchical workflow structures stored in memory, such as in a database, and presents user interface information to the service engineer necessary to carry out each service step. In a preferred embodiment, service steps are interconnected by hypertext links.

In an alternative embodiment, the service user interface may be carried out through an intranet or internet based connection with primary navigation icons being mapped to particular service delivery application starting points or associated hierarchical workflow structures and service steps.

Embodiments of the invention will now be described, by way of example, with reference to the accompanying drawings, in which:
Figure 1 shows a block diagram of a medical diagnostic system according to a preferred embodiment of the present invention.
Figure 2 shows a schematic representation of a service workflow according to a preferred embodiment of the present invention.
Figure 3 shows a schematic representation of service information and applications according to a preferred embodiment of the present invention.
Figure 4 shows a block diagram of an alternate embodiment of a medical diagnostic system according to a preferred embodiment of the present invention.
Figure 5 shows an example of a view on the display of a medical diagnostic system corresponding to a view error log step according to a preferred embodiment of the present invention.
Figure 6 shows an example of a view on the display of a medical diagnostic system corresponding to a view detailed error information step according to a preferred embodiment of the present invention.
Figure 7 shows an example of a view on the display of a medical diagnostic system corresponding to a view diagnostic parameters and instructions step according to a preferred embodiment of the present invention.
Figure 8 shows an example of a view on the display of a medical diagnostic system corresponding to a view diagnostic results step according to a preferred embodiment of the present invention.
Figure 9 shows an example of a view on the display of a medical diagnostic system corresponding to another view diagnostic parameters and instructions step according to a preferred embodiment of the present invention.
Figure 10 shows an example of a view on the display of a medical diagnostic system corresponding to another view diagnostic results step according to a preferred embodiment of the present invention.

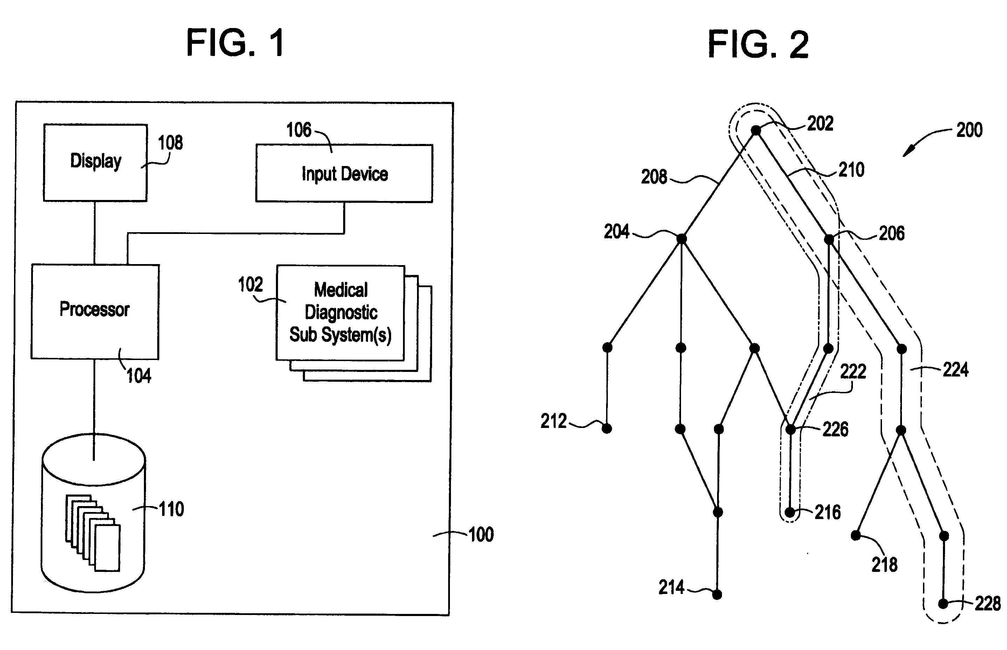

FIG. 1 illustrates a medical diagnostic system 100. The medical diagnostic system 100 comprises one or more medical diagnostic subsystems 102, a processor 104, an input device 106, a display 108 and a storage medium 110. The medical diagnostic system 100 may from time to time detect and report system errors that occur during operation to the processor 104. The processor 104 maintains a log of such system errors on storage medium 110. A user of the medical diagnostic system (which may include a service engineer) may view the display 108 and enter commands and selections using the input device 106. The storage medium 110 contains data that may represent service information and applications for use in the service process. The storage medium may be RAM, ROM, a hard drive, a CD-ROM drive, or any other medium capable of storing data. The processor 104 may access the error log and the service information and applications from time to time.

Typically, when a service engineer, either a field engineer or a remote service engineer, begins to service the medical diagnostic system 100, the service engineer first examines the error log. In the preferred embodiment, the processor 104 retrieves the error log from the storage medium 110 and prepares a formatted error log containing hypertext links associated with each error. The formatted error log may be output to the display 108 by way of a hypertext viewer application.

When formatting the error log, the processor 104 may apply one or more filters in order to sort the errors by various characteristics. By way of example, these characteristics may include time of occurrence of an error, severity of the error and the like. The filtered error log may be used by the service engineer to determine more quickly possible root causes for the errors contained in the error log. The system may apply a default filter to the log. Alternatively, the service engineer may decide which, if any, filter should be applied.

Once the formatted error log is displayed, the service engineer may use the input device 106 to select a hypertext link associated with a particular error. Selection of a hypertext link prompts the processor 104 to bring up the first service step of a service workflow associated with that error. When a plurality of service workflows are associated with the chosen error, the processor 104 presents to the service engineer an equal plurality of workflows. The service engineer may choose to follow any one of the workflows associated with the chosen error. When such multiple workflows exist, the processor 104 may recommend to the service engineer a suggested order to try the workflows. For example, the system may suggest that the service engineer first follow a workflow leading to a cause that is a probable root cause (based on historical service information). As another example, the system may suggest a particular workflow based on contextual information. This contextual information may include other errors that occur at or about the same time as the selected error. For example, the suggested workflow may depend on the exact sequencing of the errors that immediately precede the selected error in the error log.

A service workflow is a collection of one or more service paths that leads to the identification of the possible causes, and elimination of the root causes, of an error. A root cause is a true cause of an error. All of an error's root causes must be eliminated in order to stop the error from recurring. There may be multiple possible causes associated with an error. Furthermore, there may also be multiple root causes for any given error. Put another way, the service engineer may need to perform multiple tasks in order to stop the error from recurring. There may be multiple service paths through the workflow between an error and any one possible cause for the error. The service paths in a workflow may intersect with each other at various service steps. Put another way, a service step may belong to more than one service path. Furthermore, service paths and service steps may belong to multiple workflows.

There are may be different types of service steps. One type of service step guides the service engineer through the performance of one or more diagnostic tests. Diagnostic tests may involve running software diagnostic applications. The system may run software diagnostic applications automatically and communicate the results to the user. In some instances, the service engineer may have to perform a particular action before a test is run. For example, the engineer may have to attach a loopback connector to a communications interface before running the diagnostic application. In some instances, the service engineer may be given a choice of parameters that will be used to determine how the test is run. The system may suggest defaults values based on historical service information or other values based on contextual information.

Another possible type of service step is a replacement step. During a replacement step, the system guides the service engineer through the procedures necessary to replace a part of the medial diagnostic system. If the replacement part is not readily available, then the system may provide the service engineer with the information necessary to order the part. A replacement step may also collect parts utilization information from the engineer. For example, the system may ask the service engineer if replacing a given part solved the problem. Parts utilization information may be used to help improve service part inventory control. Parts utilization information may also be used to improve the context sensitivity of the service system by providing historical data that the method may use to select one of multiple branches in a workflow when the need arises.

Another type of service step offered by the preferred embodiment is a recalibrating step. At a recalibrating step, the system guides the service engineer through the recalibration of a part of the medical diagnostic system. For example the service engineer may be guided through the recalibration of an imaging subsystem.

The preferred embodiment may also include service steps to keep track of the amount of time spent servicing the system (for evaluation or accounting purposes). The system may also provide service steps that help to maintain a schedule of preventative maintenance for the medical diagnostic system.

A service engineer may follow a service path through a workflow to its end and then determine that the workflow has lead to a non-root cause. In other words, he has reached a possible cause that is in fact not the cause of an error occurrence. When this occurs, the service engineer may traverse back through the path of service steps to any point at which there was a choice as to which of multiple service paths to follow. The service engineer may then follow another service path. Backtracking may be achieved by using the hypertext viewer to traverse back through the various service steps executed. The method may automatically keep track of which paths have been followed. If a workflow does not lead to isolation and elimination of a root cause, then the service engineer may try an alternative workflow in the same manner. In other words, the service engineer may traverse back through the path of service steps to any point at which there was a choice as to which of multiple workflows to follow. The service engineer may then follow another workflow.

FIG. 2 illustrates a schematic representation of a hierarchical workflow structure 200. The hierarchical workflow structure 200 has an initial service step 202. The initial service step 202 is connected to intermediate service steps 204 and 206 by service path segments 208 and 210, respectively. With respect to a hierarchical workflow structure, an intermediate service node is any service node that is neither an initial service node nor a final service node. The many intermediate service steps define a number of service paths between the initial service step 202 and the final service steps 212, 214, 216, 218 and 220. Service paths 222 and 224 extend from the initial service step 202 to the final service steps 216 and 220, respectively. A service step may be located on multiple service paths. For example, the service step 206 is common to the service path 222 and the service path 224. Since the service paths 222 and 224 are identical until service step 206, these service paths are said to branch at service step 206. Further, as shown at service step 226, two service paths may join together at a service step. A complete service path connects an initial service node to a final service node. However, a service path is any subset, in sequence, of service steps within such a complete service path. It is important to note that the initial service step 202 may be an application starting point. An initial service step 202 may also be a step associated with an error in the error log. In some instances, an initial service step 202 may be a final service step of another service workflow. In this way, a workflow may be linked to other workflows. The system may contain many workflows, and these workflows may share common service steps. Put another way, the workflows within the system may overlap.

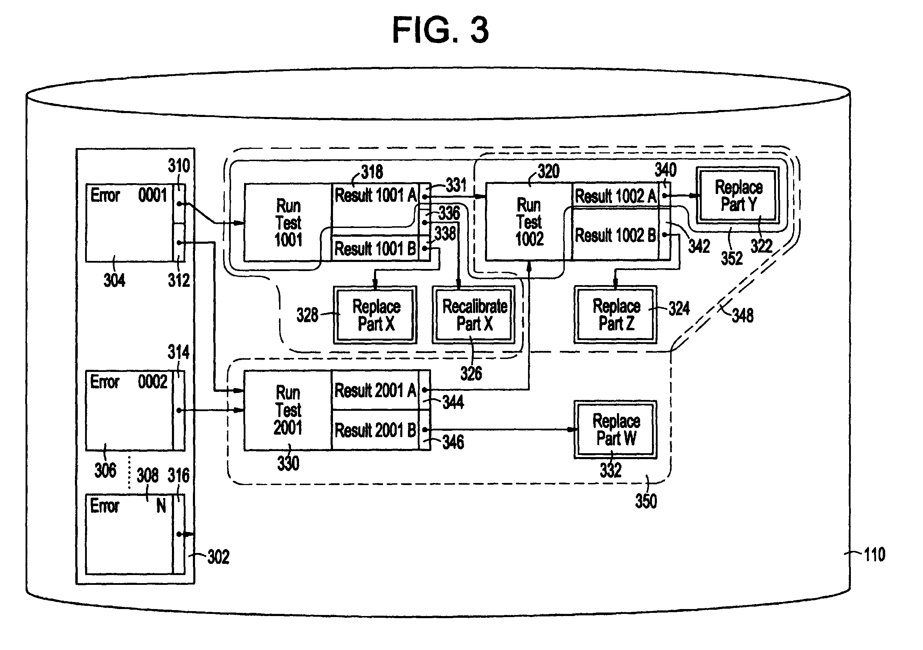

FIG. 3 illustrates a schematic representation of service information and data as stored on the storage medium 110. FIG. 3 shows a formatted error log 302 recorded by the system and displayed to the service engineer. The errors may be captured and logged by the system during normal operation. Each of the errors 304, 306 and 308 has one or more hypertext links assigned to it based on historical performance. The hypertext links 310 and 312 are associated with the error 304. The hypertext links 314 and 316 are associated with the errors 306 and 308, respectively. FIG. 3 also shows a number of service steps 318, 320, 322, 324, 326, 328, 330 and 332. Some of the service steps are interconnected with each other via the hypertext links 334, 336, 338, 340, 342, 344, 346. The hypertext links 310, 312, 314 and 316 are predetermined based on prior knowledge of operation of the system. The hypertext links 310, 312, 314 and 316 are stored in the service workflow storage medium 110. When the system detects an error and places it on the formatted error log 302, the storage medium 110 is accessed to identify any associated service steps. In the example of FIG. 3, the service steps 318, 320, 322, 324, 326, 328, 330 and 332 are identified and associated hypertext links are constructed.

The service steps 318, 320, 322, 324, 326 and 328, together with the hypertext links 334, 336, 338, 340, and 342, comprise a service workflow 348 associated with the error 304 by the hypertext link 310. The service steps 320, 322, 324, 330 and 332, together with the hypertext links 340, 342, 344, and 346, comprise a service workflow 350. Service workflow 350 is associated with the error 304 by the hypertext link 312. Service workflow 350 is also associated with the error 306 by the hypertext link 314.

There are multiple possible service paths through the service workflow 350. For example, the service path 352 through the service workflow 350 begins with the service step 318, continues through the hypertext link 334, the service step 320, the hypertext link 340 and ends with service step 322. If the service engineer follows the service path 352 but does not prevent the recurrence of the error 304, the service engineer may traverse back through the service path 352 to the service step 318. The service step 318 was the last point at which the service engineer made a decision as to which of multiple possible service paths through the workflow to follow. The service engineer may then follow the hypertext link 336 to service step 326.

In a medical diagnostic system, there may be hundreds of possible errors and thousands of possible service steps. The service steps may be interconnected in many different permutations and combinations providing for a very large number of possible service paths and workflows. The preferred embodiment tracks, for the service engineer, the context in which service steps are performed. The system may also use contextual tracking information to suggest a service path or a workflow whenever a choice to follow one of them must be made. For example, the system may suggest a first possible service path over a second if a particular service step has already been performed. Also, the system may suggest one possible workflow sequence over another if a third workflow sequence has already been carried out, but did not solve an error. As a third example, the system may suggest one of multiple possible service paths based on the results of a diagnostic step already performed as part of another workflow sequence.

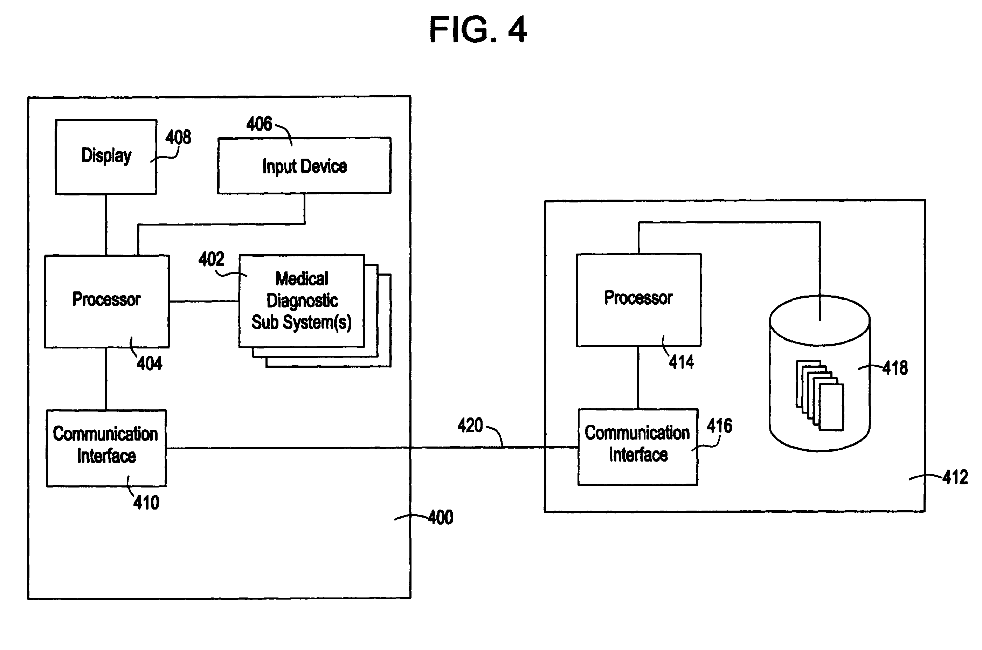

In an alternative embodiment of the present invention, shown in FIG. 4, the service information, applications or both may be stored remotely. FIG. 4 illustrates a medical diagnostic system 400. The medical diagnostic system 400 comprises one or more medical diagnostic subsystems 402, a processor 404, an input device 406, a display 408 and a communications interface 410. FIG. 4 also depicts a service server 412. The service server 412 comprises a processor 414, a communications interface 416 and a storage medium 418.

Service delivery for the medical diagnostic system 400 is accomplished in a similar manner to the medical diagnostic system 100 (as discussed above). However, when being serviced by a service engineer, the medical diagnostic system 400 must connect to the service server 412 in order to access any service information or applications which are stored on the storage medium 418. This may be accomplished using a communications link 420 between the communications interfaces 410 and 416. The communications link 420 link may be any carrier suitable for the transmission of data. For example, the communications link 420 may be a proprietary telephone link, in which case the communications interfaces 410 and 416 may be modems. The communications link 420 may also be the internet, in which case the communications interfaces 410 and 416 may be internet adapter cards (ethernet cards, for example). Other types of communications links (ISDN, cellular, radio, satellite, direct wire, and others) may be used, along with their corresponding communications interfaces.

When connected to the service server 412, the medical diagnostic system 400 may access service information and applications stored there on the storage medium 418. Further, up to date parts inventory information may be obtained. The service server 412 may be configured to accept parts orders from the service engineer.

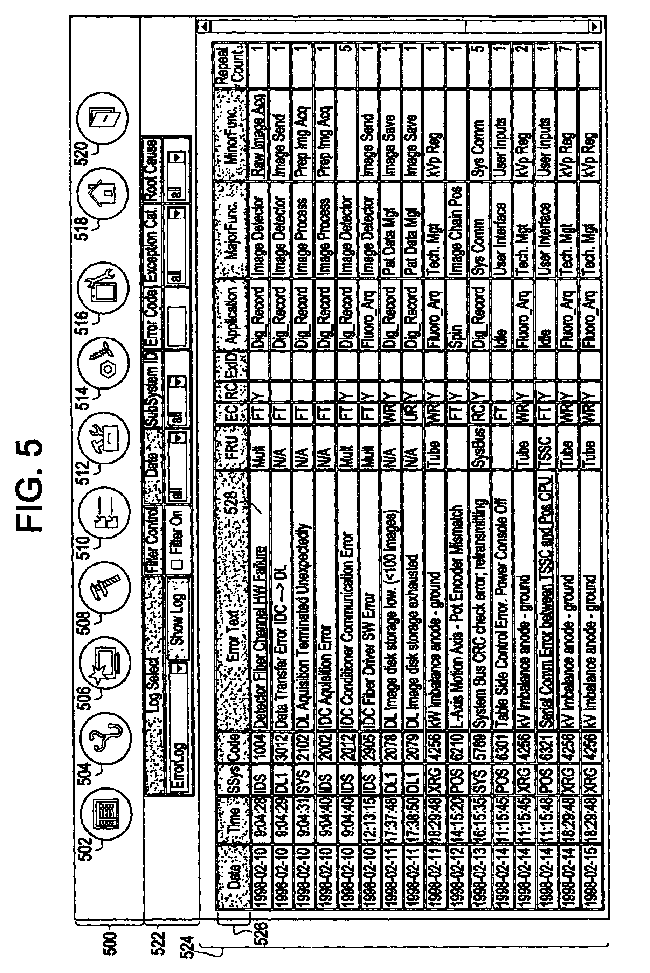

An example of a screen from the display of a medical diagnostic system implementing an embodiment of the present invention is shown in FIG. 5. Located at the top portion of the screen is a navigation bar 500. The navigation bar 500 comprises the icons 502, 504, 506, 508, 510, 512, 514, 516, 518 and 520. Each of the icons 502, 504, 506, 508, 510, 512, 514 and 516, is associated with a main service application. Shown in FIG. 5 are icons corresponding to a error log viewer application 502, a diagnostic application 504, an image quality application 506, a calibration application 508, a configuration application 510, a toolbox/utilities application 512, a spare parts viewer application 514, and a preventative maintenance application 516. The user may select the home icon 518 to return to a home screen of the service application. The user may exit the service application by selecting the exit icon 520. A filter control 522 is located in the middle portion of the screen. The user may use the filter control 522 to filter the error log by various parameters. For example, the user may apply a filter to display only those errors that occurred within a particular period. Alternatively, the user could apply a filter to display only those errors that are of the greatest severity.

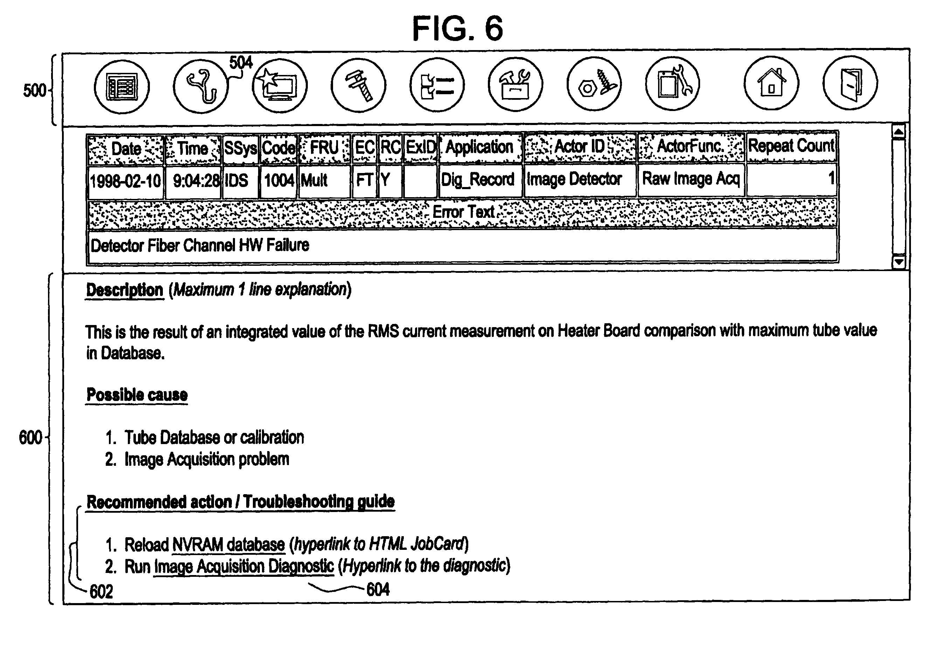

In FIG. 5, the lower portion of the display contains the error log viewer 524. In the example of FIG. 5, the user of the system had selected the view error log application icon 502 from the navigation bar 500. The error log viewer 524 may display the date and time an error occurred, a text description of the error, the subsystem that recorded the error, an internal error code, their severity of the error, and other useful information. From the error log viewer 524, the user may select a specific error and be shown more detailed information about the error. In this example, the user may select the error 526 by selecting the hypertext link 528. FIG. 6 shows an example of a screen that displays detailed error information to the user about the error 526.

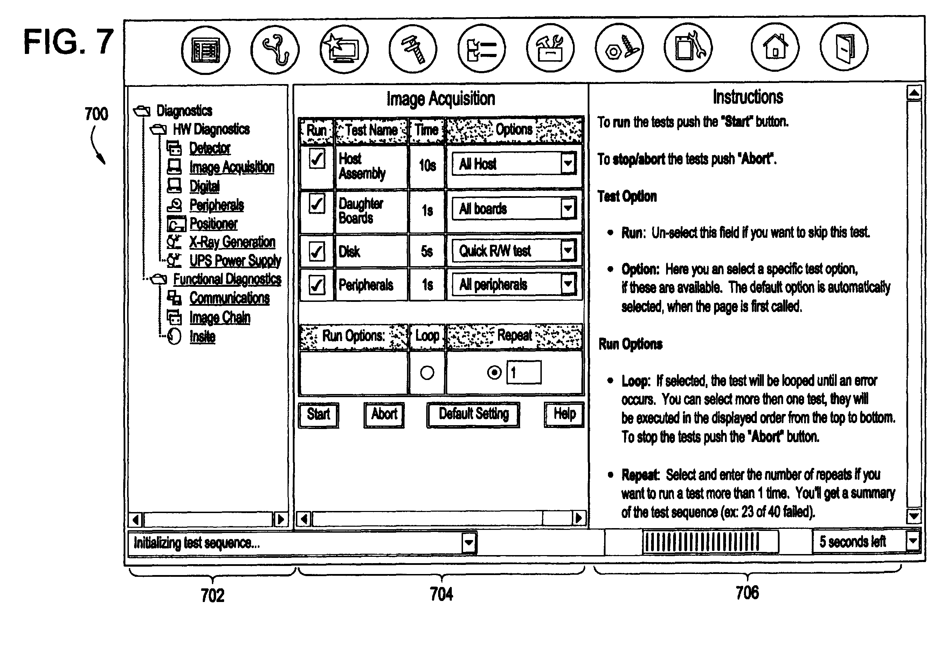

As seen in FIG. 6, the detailed error information 600 includes one or more recommended service workflows 602. One of these recommended workflows may be to run a particular diagnostic test, as is the case here. At this time, the user may select a particular hypertext link associated with one of the recommended service workflows 602. The system may then automatically launch the diagnostic application to analyze specifically the part or subsystem associated with the selected workflow. Alternatively, the user may use the navigation bar 500 to bring up the diagnostic application (by selecting the navigation icon 504) and navigate to the diagnostics for the related subsystem or part. The direct hypertext link facilitates quicker service and eliminates possible user error. In this example, the user may select a hypertext link 604 to run the diagnostics for the image acquisition subsystem (which is the sub system containing the suspected failed component). Once the user selects the hypertext link 604, the system runs the diagnostic application and the display will show the diagnostic parameters and instructions view 700, as in FIG. 7.

The lower portion of the screen 700 in the initial view of the diagnostic application comprises three areas. The leftmost area 702 shows a tree structure corresponding to the arrangement of subsystems and parts within the medical diagnostic system. The center area 704 shows the parameters, if any, for the test about to be run for the currently selected subsystem or part. If there are parameters for the test that the service engineer may adjust, then the engineer may adjust those parameters in the center area 704. The rightmost area 706 of the display shows instructions for the test (including, for example, an explanation of any adjustable test parameters). The system may suggest appropriate default or other values for the test, based on historical service information and context sensitivity. If a test is automated (i.e., it requires no user intervention) then this area will inform the engineer of that fact.

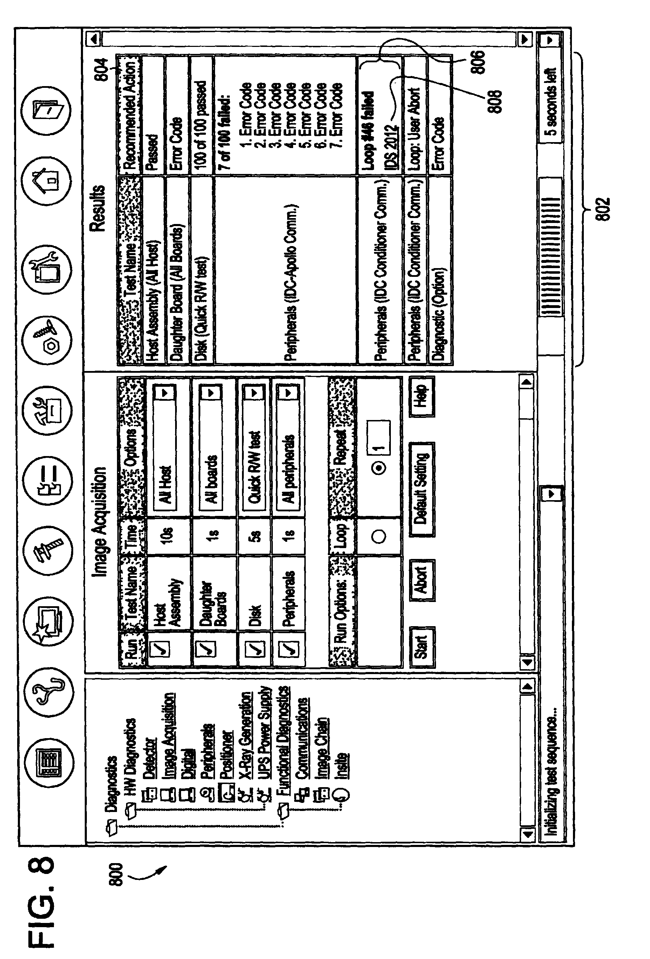

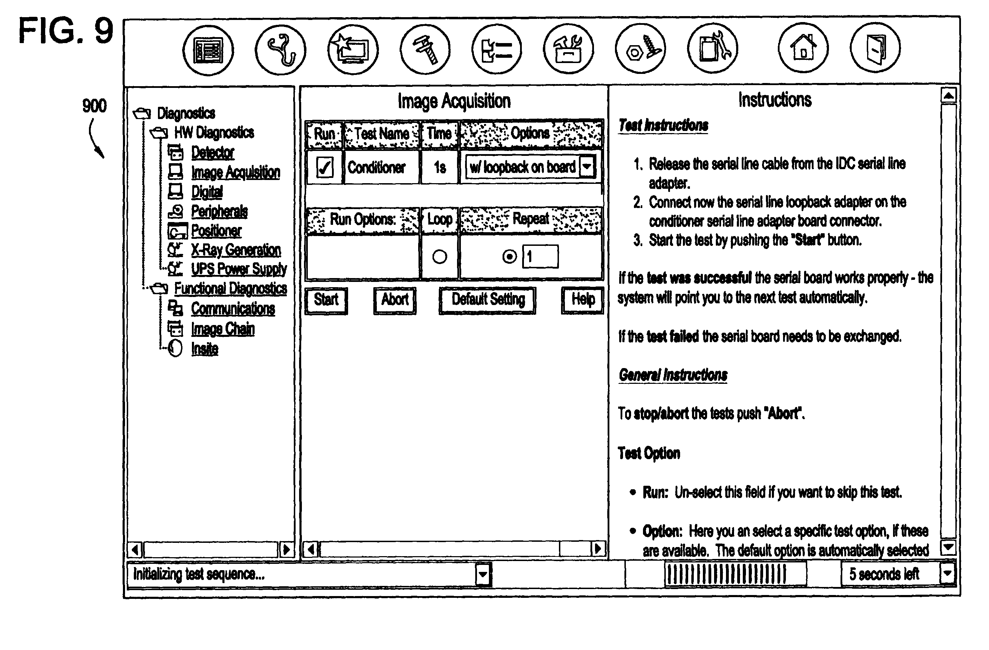

FIG. 8 shows an example of a diagnostic results view 800. This view is shown after the diagnostic test is completed. A results area 802 replaces the instruction area 702 (see FIG. 7) in the rightmost area of the view 800. The results of the test that has just been run are shown in the results area 802. The results area 802 also shows recommended actions 804 based on the results of the diagnostic test. In the present example, the fifth result 806 contains a hypertext link 808 to another diagnostic test. Once the user selects the hypertext link 808, the system switches to this second diagnostic test. The system will then display the diagnostic parameters and instructions view 900 for the second test, as shown in FIG. 9.

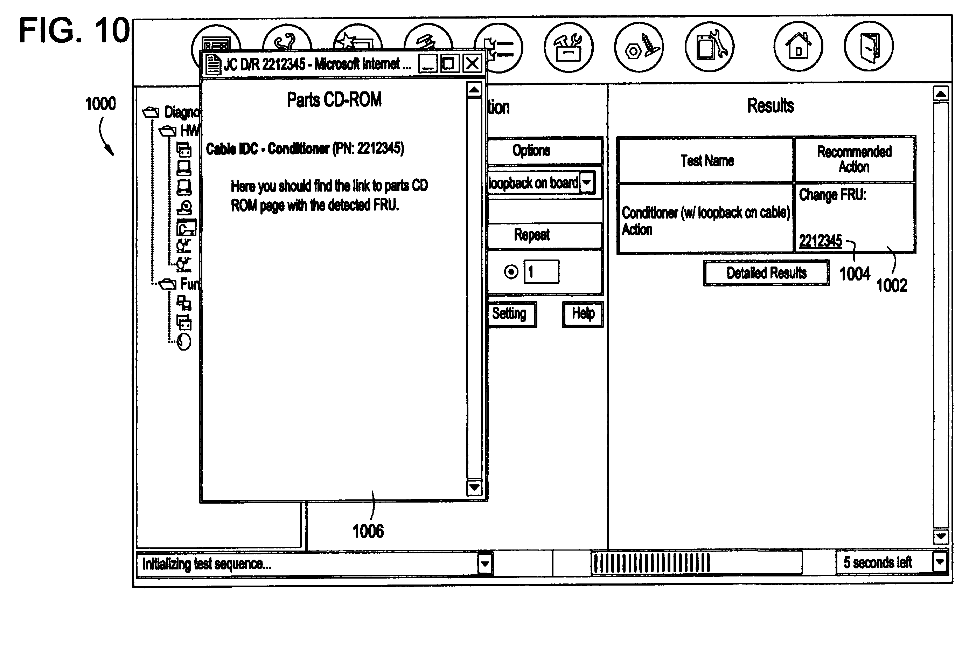

After the second diagnostic test has been run, the results of the second diagnostic test are displayed. FIG. 10 shows the second diagnostic results view 1000. The result of the second diagnostic test instructs the user, at 1002, to replace a part of the system. This result also contains a hypertext link 1004. By selecting the hypertext link 1004, the engineer may access a database of parts inventory and part ordering information. This database may either be accessed from a storage media (such as a CD-ROM) or from a server to which the medical diagnostic system is connected. The information concerning the part to be replaced may be shown in a separate viewer window 1006, as shown in FIG. 10. This information may also include a description of the procedures necessary to replace the part.

If the test shown in FIG. 9 had shown positive results, then there would be no recommended replacement action. In this case, the system would recommend that the user go back to search for other possible causes of the error. The user would use the hypertext browser to step back to the last point at which there was a branch. In this example, that would be at the view of results of the first diagnostic test 800, shown in FIG. 8.

The preferred embodiments provide for a consistent service platform by using a hypertext viewer that may be used across different products and product lines. While the service information and software applications will often be product specific, they will be presented with a similar interface via the hypertext viewer. The preferred embodiments further provide for an automatic way to keep the context for and guide the service engineer through the service delivery process. The preferred embodiments also provide for a service delivery method and apparatus that allows for easy updating of service information and applications by storing these on a centralized service server.

## Claims

1. A method for automatically guiding a user through a service workflow (348) of a medical diagnostic system (100, 400), the method comprising:
automatically cataloging a system error (304) that occurred during operation of the medical diagnostic system (100, 400) into an error log (302);
generating from the error log (302) a hypertext link (310)
corresponding to a first service step (318) of a service workflow (348) associated with the system error (304); and
guiding the user through a service workflow (348) to eliminate a cause of the system error.

2. The method of claim 1 wherein the step of generating further comprises applying a filter to the error log.

3. The method of claim 1 wherein the guiding step comprises guiding the user through execution of a system diagnostic application, or through replacement of a part of the medical diagnostic system (100, 400).

4. The method of claim 1 further comprising connecting the medical diagnostic system (400) to a server (412) and downloading a system diagnostic application or ordering a replacement part through the server (412).

5. The method of claim 1 wherein the guiding step comprises guiding the user through recalibration of a part of the medical diagnostic system (100, 400).

6. The method of claim 1 wherein the guiding step further comprises guiding the user through a document containing hypertext links.

7. The method of claim 6 wherein the guiding step further comprises connecting the medical diagnostic system (400) to a server (412) and downloading the document from the server (412).

8. The method of claim 1 wherein the guiding step further comprises providing a set of possible service paths (222, 224) to the user, stemming from a current service step (202), or based on contextual information, or based on historical service data.

9. The method of claim 1 further comprising providing the user with a navigational interface (500).

10. A medical diagnostic system (100) implementing a method for automatically guiding a user through a service workflow (348) of the medical diagnostic system (100), comprising:
a medical diagnosis subsystem (102);
a storage medium (110) for storing an automatic catalog of a system error (304) that occurred during operation of the medical diagnostic system (100) in an error log (302);
a processor (104) generating from the error log (302) a hypertext link (310) corresponding to a first service step (318) of a service workflow (348) associated with the system error (302); and
a display (108) visually guiding a user through a service workflow (348) to eliminate a cause of the system error (302).

11. The medical diagnostic system (400) of claim 10 further comprising a communications interface (410) for connecting the medical diagnostic system (400) to a remote system (412) over a communications link (420), the processor (404) receiving and sending service information over the communications link (420).

12. The medical diagnostic system (400) of claim 11 wherein the communications link (420) is a proprietary telephonic dial up or is the internet.

13. The medical diagnostic system (100) of claim 10 wherein the storage medium (110) stores a hierarchical workflow structure (200) and the processor (104) generates the workflow (348) from the hierarchical workflow structure (200).
